# EUROPEAN PATENT SPECIFICATION

(11) **EP 2 712 858 B1**
(45) Date of publication and mention of the grant of the patent: **07.03.2018**
(21) Application number: 13184535.6
(22) Date of filing: 16.09.2013
(51) Int. Cl.: C07C 327/26

(54) **Process for producing thionocarboxylic acid ester**
Verfahren zur Herstellung von Thionocarboxylsäureester
Procédé de production d'ester d'acide thiono-carboxylique

(30) Priority: 27.09.2012 JP 2012213968
(43) Date of publication of application: 02.04.2014
(73) Proprietor: Daikin Industries, Ltd., Osaka-shi, Osaka 530-8323 (JP)
(72) Inventor: Namikawa, Takashi, Osaka, 566-0044 (JP); Kishikawa, Yousuke, Osaka, 566-0044 (JP); Nomura, Takashi, Osaka, 566-0044 (JP); Tanaka, Asako, Osaka, 566-0044 (JP); Adachi, Kenji, Osaka, 566-0044 (JP)
(74) Representative: Hoffmann Eitle

(56) References cited:
- HORST VIOLA ET AL: "Organische Schwefelverbindungen,97. Friedel-Crafts-Reaktionen mit Thiosäurechloriden", CHEMISCHE BERICHTE, vol. 101, no. 10, 1 October 1968 (1968-10-01), pages 3517-3529, XP55084572, ISSN: 0009-2940, DOI: 10.1002/cber.19681011024
- KLAUS HARTKE ET AL: "[alpha],[beta]-acetylenic dithio and thiono esters", TETRAHEDRON LETTERS, vol. 30, no. 9, 1 January 1989 (1989-01-01), pages 1073-1076, XP55084574, ISSN: 0040-4039, DOI: 10.1016/S0040-4039(01)80363-4
- J. R. FALCK ET AL: "Tin-Copper Transmetalation: Cross-Coupling of .alpha.-Heteroatom-Substituted Alkyltributylstannanes with Organohalides", JOURNAL OF THE AMERICAN CHEMICAL SOCIETY, vol. 117, no. 22, 1 June 1995 (1995-06-01) , pages 5973-5982, XP55084573, ISSN: 0002-7863, DOI: 10.1021/ja00127a010
- C. CARDELLICCHIO ET AL: "A highly efficient synthetic route to ketones through sequential coupling reactions of grignard reagents with -phenyl carbonochloridothioate in the presence of nickel or iron catalysts", TETRAHEDRON LETTERS, vol. 26, no. 30, 1 January 1985 (1985-01-01), pages 3595-3598, XP55084505, ISSN: 0040-4039, DOI: 10.1016/S0040-4039(00)89199-6

## Description

### Technical Field

The present invention relates to a process for producing an ester of aryl-containing thionocarboxylic acid that is useful as, for example, a liquid crystal material.

### Background Art

The aryl ester of arylthionocarboxylic acid of the formula R¹C=(S)OR², wherein R¹ and R² independently are an optionally substituted aryl group, is a compound that is useful as, for example, a liquid crystal material.

Various methods are known as methods for producing thionocarboxylic acid esters having this structure. Examples of such methods include a method comprising reacting a chlorothionoformic acid ester with an aromatic compound ((1): H. Viola, et al., Chem. Ber., 101, 3517 (1968); a method comprising treating a carbanion with a dithiocarbonic acid ester or a thionocarbonic acid ester ((2): Liebigs Ann. Chem., 1973, 1637); a method comprising reacting an orthoester with hydrogen sulfide ((3): A. Ohno et al., Tetrahedron Lett., 1968, 2083); a method comprising reacting a carboxylic acid ester with phosphorus pentasulfide or Lawesson's reagent ((4): Synthesis, 1973, 149 and (5): Bull. Chem. Soc. Belg., 87, 293 (1987)); a method comprising reacting a thionocarboxylic acid chloride with an alcohol or phenol ((6): S. Scheithauer et al., Chem. Ber., 98, 838 (1965)); a method comprising reacting a nitrile with an alcohol and then reacting the resulting product with hydrogen sulfide ((7): Liebigs Ann. Chem., 1974, 671); a method comprising treating a methylated aromatic compound with sulfur and an alcohol ((8): Z. Chem., 6, 108 (1966)); and a method comprising reacting a thioacyl disulfide with an alcoholate ((9): K.A. Latif et al., Tetrahedron, 26, 4247 (1970)).

However, among these methods, the methods disclosed in, for example, (1), (2), (4), (5) and (8) are low-yield; in particular, the method disclosed in (4) and (5) requires a high temperature of 100°C or more for the reaction, and also has a drawback of generating a large amount of by-products; therefore, these methods cannot be regarded as efficient methods.

The methods disclosed in (3) and (7) use hydrogen sulfide, which is so toxic that its handling requires specialized equipment; therefore, these methods are unsuitable as industrial processes for production. Further, the method disclosed in (6) cannot be regarded as a comprehensively superior method due to a low synthesis yield of the starting thionocarboxylic acid chloride. The method disclosed in (9) comprises a large number of steps, including synthesizing a thioacyl disulfide from a dithiocarboxylic acid derivative and then reacting the thioacyl disulfide with an alcoholate; therefore, this method cannot be regarded as a convenient method.

K. Hartke et al., Tetrahedron Lett., 30(9), 1073-76 (1989) describes a process for preparing aryl esters of thionocarboxylic acid by e.g. reacting a compound of the formula Phenyl-O-C(=S)-Cl (14e) with Phenyl-C=C-Mg-Br to obtain a compound Phenyl-O-C(=S)-C=C-Phenyl (2e).

### Summary of Invention

### Technical Problem

The present invention was made in view of the above-mentioned problems in the prior art. A primary object of the invention is to provide a relatively convenient and industrially advantageous process for producing, in a high yield, an arylthionocarboxylic acid ester, which is useful as, for example, a liquid crystal material.

### Solution to Problem

To achieve the above object, the present inventors conducted extensive research. As a result, the inventors found that when a halogenated thiocarbonyl compound is used as a starting material and reacted with a Grignard compound having an aryl group or a cycloalkyl group, the desired arylthionocarboxylic acid ester can be produced in a high yield under mild reaction conditions using relatively safe starting materials. The present invention has been accomplished based on this finding.

More specifically, the present invention provides a process for producing an arylthionocarboxylic acid ester of formula (3) :

R-C(=S)-O-Ar (3)

wherein Ar is optionally substituted aryl, and R is aryl or cycloalkyl, each optionally substituted; comprising reacting a halogenated thiocarbonyl compound of formula (1):

X-C(=S)-O-Ar (1)

wherein Ar is as defined above and X is halogen, with a Grignard compound of the formula R-MgY (2) wherein R is as defined above and Y is halogen, in the presence of at least one component selected from trivalent iron compounds, monovalent copper compounds, divalent copper compounds, divalent nickel compounds, divalent zinc compounds, and divalent cobalt compounds.

Also, the invention provides a process for producing an arylthionocarboxylic acid ester of formula (3):

R-C(=S)-O-Ar (3)

wherein Ar is an optionally substituted aryl group and R is optionally substituted aryl or optionally substituted cycloalkyl; the process comprising reacting a halide represented by Formula R-Y (4) wherein R is as defined above and Y is a halogen atom, with a magnesium compound, and then reacting the reaction product with a halogenated thiocarbonyl compound of formula (1):

X-C(=S)-O-Ar (1)

wherein Ar is as defined above and X is a halogen atom, in the presence of at least one component selected from trivalent iron compounds, monovalent copper compounds, divalent copper compounds, divalent nickel compounds, divalent zinc compounds, and divalent cobalt compounds.

Preferred embodiments of the present invention are as defined in the appended dependent claims and/or in the following detailed description.

The process of the present invention for producing an arylthionocarboxylic acid ester is explained in more detail below.

The process of the present invention for producing an arylthionocarboxylic acid ester comprises reacting a halogenated thiocarbonyl compound X-C(=S)-O-Ar (1) wherein Ar is optionally substituted aryl and X is halogen, with a Grignard compound R-MgY (2) wherein R is aryl or cycloalkyl, each optionally substituted and Y is halogen, in the presence of at least one component selected from trivalent iron compounds, monovalent copper compounds, divalent copper compounds, divalent nickel compounds, divalent zinc compounds, and divalent cobalt compounds. This process can produce an arylthionocarboxylic acid ester R-C(=S)-O-Ar (3) wherein Ar and R are as defined above, in a high yield under relatively mild conditions.

First, the starting compounds used in the production process of the present invention are explained in more detail.

### Starting compounds

### (1) Halogenated thiocarbonyl compound

In the starting halogenated thiocarbonyl compound (1), Ar is an optionally substituted aryl group. Examples of optionally substituted aryl groups include optionally substituted phenyl and optionally substituted naphthyl.

There is no particular limitation on the substituent on the aryl group, insofar as the substituent is inert to the reaction with a Grignard compound R-MgY (2) described below. Specific examples of such substituents include alkyl, fluoroalkyl, alkoxy, fluoroalkoxy, cyano, aryl and halogen. Among these, examples of alkyl groups include linear or branched C₁₋₅-alkyl; and examples of fluoroalkyl groups include linear or branched C₁₋₅-alkyl in which hydrogen atoms are partially or completely replaced by fluorine. Examples of alkoxy groups include linear or branched C₁₋₅-alkoxy. Examples of fluoroalkoxy groups include linear or branched C₁₋₅-alkoxy in which hydrogen atoms are partially or completely replaced by fluorine. Examples of aryl groups as substituents include phenyl and naphthyl. The substituent aryl groups may further contain a substituent that is inert to the reaction. Examples of halogen atoms include F, Cl, Br and I.

In the above formula (1), the halogen atom represented by X may be, for example, F, Cl, Br or I. Chlorine is particularly preferable.

### (2) Grignard compound

In the Grignard compound R-MgY (2) wherein R is an aryl or cycloaryl, each optionally substituted, and Y is a halogen atom, the optionally substituted aryl group represented by R may be, for example, optionally substituted phenyl or optionally substituted naphthyl. The substituent on the aryl group may be any substituent that is inert to the reaction with a halogenated thiocarbonyl compound (1), and that is also inert to the reaction for producing a Grignard compound R-MgY (2) from a halide R-Y (4) described below. Specific examples of such substituents may be the same as the aforementioned examples of substituents on the aryl group represented by Ar.

The cycloalkyl group of the optionally substituted cycloalkyl group represented by R may be, for example, a C₃₋₈-cycloalkyl group, and preferably C₅₋₇-cycloalkyl. Examples of such cycloalkyl groups include cyclopropyl, cyclobutyl, cyclopentyl, cyclohexyl, cycloheptyl and cyclooctyl. The substituent on the cycloalkyl group may be any substituent that is inert to the reaction with a halogenated thiocarbonyl compound (1), and that is also inert to the reaction for producing a Grignard compound R-MgY (2) from a halide R-Y (4) described below. Specific examples of such substituents include alkyl, cycloalkyl and fluoroalkyl. Among such substituents, examples of alkyl groups include linear or branched C₁₋₅-alkyl; and examples of fluoroalkyl groups include linear or branched C₁₋₅-alkyl in which hydrogen atoms are partially or completely replaced by fluorine. Examples of cycloaryl groups as substituents include C₃₋₈-cycloalkyl. The substituent cycloaryl groups may further contain a substituent that is inert to the reaction, such as alkyl.

To obtain a Grignard compound R-MgY (2), for example, a halide R-Y (4) may be reacted with a magnesium compound.

The magnesium compound that can be used in this reaction is not particularly limited insofar as the compound is reactive with a halide (4) to produce a Grignard compound. Specific examples of such magnesium compounds include metal magnesium, i-PrMgCl (isopropylmagnesium chloride), i-PrMgBr (isopropylmagnesium bromide), and an i-PrBu₂MgLi ate complex which can be produced from n-BuLi and i-PrMgCl or i-PrMgBr.

The amount of the magnesium compound may be 1.0-10.0 mol, and preferably 1.0-2.0 mol, per mol of the halide R-Y (4).

Typically, the reaction of a halide R-Y (4) with a magnesium compound is preferably performed in an aprotic organic solvent inert to the reaction. Examples of aprotic organic solvents include tetrahydrofuran, diethyl ether, 2-methyltetrahydrofuran, dibutyl ether, tert-butyl methyl ether, cyclopentyl methyl ether, dimethoxyethane, dioxane, benzene, toluene, xylene, pentane, hexane and heptane. Such aprotic organic solvents may be used as a mixture of two or more, if necessary.

There is no particular limitation on the concentrations of the starting materials in the reaction solvent. For example, the concentration of the halide R-Y (4) may be 0.5-10.0 mol/l.

The temperature at which the reaction of a halide R-Y (4) with a magnesium compound is performed is preferably 0-100°C, and more preferably 20-70°C.

The pressure during the reaction is not particularly limited, and can typically be performed under atmospheric pressure. The atmosphere during the reaction is not particularly limited. Typically, the reaction is preferably performed in an inert gas atmosphere, such as a nitrogen atmosphere or an argon atmosphere. Typically, the reaction time may be 30 minutes to 10 hours.

The yield of the Grignard compound R-MgY (2) can be increased by reacting a halide R-Y (4) with a magnesium compound in the presence of, for example, LiCl, iodine or dibromoethane. The total amount of these ingredients is preferably 1.0-5.0 mol, and more preferably 1.0-1.2 mol, per mol of the halide R-Y (4).

The above process can produce a Grignard compound R-MgY (2) wherein R is an aryl or cycloalkyl,, each optionally substituted, and Y is a halogen atom.

### Process for producing an arylthionocarboxylic acid ester

As described above, the desired arylthionocarboxylic acid ester R-C(=S)-O-Ar (3) wherein Ar is optionally substituted aryl, and R is aryl or cycloalkyl, each optionally substituted, can be obtained by reacting a halogenated thiocarbonyl compound X-C(=S)-O-Ar (1) wherein Ar is optionally substituted aryl and X is halogen, with a Grignard compound R-MgY (2) wherein R is as defined above and Y is halogen, in the presence of at least one component selected from trivalent iron compounds, monovalent copper compounds, divalent copper compounds, divalent nickel compounds, divalent zinc compounds, and divalent cobalt compounds.

The amount of the Grignard compound R-MgY (2) wherein R and Y are as defined above may be 0.8-2.0 mol, and preferably 0.9-1.2 mol, per mol of the halogenated thiocarbonyl compound (1).

The reaction of a halogenated thiocarbonyl compound (1) with a Grignard compound R-MgY (2) is preferably performed in an aprotic organic solvent. Examples of usable aprotic organic solvents may be the same as the aforementioned examples of solvents that can be used in the reaction of a halide R-Y (4) with a magnesium compound.

There is no particular limitation on the concentrations of the starting materials in the reaction solvent. For example, the concentration of the halogenated thiocarbonyl compound (1) may be 0.1-3.0 mol/l.

The temperature at which the reaction of a halogenated thiocarbonyl compound with a Grignard compound is performed is such that when using a Grignard compound wherein R is an optionally substituted aryl group, it is preferably -78 to 25°C, and more preferably -50 to 0°C; and when using a Grignard compound wherein R is an optionally substituted cycloalkyl group, it is preferably -78 to 0°C, and more preferably -78 to -30°C.

The pressure during the reaction is not particularly limited. The reaction can typically be performed under atmospheric pressure. The atmosphere during the reaction is not particularly limited. Typically, the reaction is preferably performed in an inert gas atmosphere, such as a nitrogen atmosphere or an argon atmosphere. Typically, the reaction time may be 0.5-10.0 hours.

In the production process of the present invention, the yield of the desired arylthionocarboxylic acid ester (3) is increased by reacting a halogenated thiocarbonyl compound (1) with a Grignard compound R-MgY (2) in the presence of a catalyst selected from trivalent iron compounds, monovalent copper compounds, divalent copper compounds, divalent nickel compounds, divalent zinc compounds and divalent cobalt compounds. Such compounds can be used singly, or as a mixture of two or more. Specific examples of trivalent iron compounds include tris(2,4-pentanedionato)iron(III) and ferric chloride. Examples of monovalent or divalent copper compounds include copper halides such as copper (I) chloride, copper(I) bromide, and copper (I) iodide; copper cyanide (CuCN), cupric chloride and dilithium tetrachlorocuprate(II) (Li₂CuCl₄). Examples of divalent nickel compounds include nickel chloride. Examples of divalent zinc compounds include zinc chloride. Examples of divalent cobalt compounds include cobalt chloride.

Such a catalyst is preferably used in an amount of 0.01-2.00 mol, and more preferably 0.01-0.10 mol, per mol of the halogenated thiocarbonyl compound (1).

The above process can produce an arylthionocarboxylic acid ester (3) in a high yield.

The arylthionocarboxylic acid ester obtained by the above process can be purified and collected by a known method, such as silica gel column chromatography or recrystallization.

In the present invention, instead of directly reacting a halogenated thiocarbonyl compound X-C(=S)-O-Ar (1) with a Grignard compound R-MgY (2), the following process can also be used to produce the desired arylthionocarboxylic acid ester (3). That is, a halide R-Y (4), which is a starting material for producing a Grignard compound R-MgY (2), is reacted with a magnesium compound and then the obtained reaction product is reacted with a halogenated thiocarbonyl compound X-C(=S)-O-Ar (1).. This method can produce the desired arylthionocarboxylic acid ester (3) in a high yield in a continuous process without separating a Grignard compound -MgY (2), which is an intermediate product. The reaction conditions in the step of reacting a halide (4) with a magnesium compound and in the step of reacting the reaction product with a halogenated thiocarbonyl compound (1) in the continuous process may be the same as the aforementioned conditions in each reaction step.

The arylthionocarboxylic acid ester obtained by this process can be purified and collected by a known method, such as silica gel column chromatography or recrystallization.

### Advantageous Effects of Invention

According to the present invention, the desired arylthionocarboxylic acid ester can be obtained in a high yield by using relatively inexpensive and highly safe starting materials under mild reaction conditions.

### Description of Embodiments

The present invention is explained in more detail below with reference to Examples.

### Example 1

Magnesium (1.6 g, 64.0 mmol), LiCl (2.7 g, 64.0 mmol), and tetrahydrofuran (hereinafter abbreviated as "THF")(10.0 mL) were added in a nitrogen atmosphere to a 50 ml three-necked flask equipped with a stirring bar and a thermometer. After chlorobenzene (7.2 g, 64 mmol) was added, the mixture was stirred at room temperature for 2 hours to produce a Grignard compound.

Subsequently, phenyl chlorothionoformate (11.0 g, 64.0 mmol), tris(2,4-pentanedionato)iron(III) (Fe(acac)₃) (0.7 g, 1.9 mmol), and THF (50.0 mL) were added in a nitrogen atmosphere to a 200 ml three-necked flask equipped with a stirring bar and a thermometer. After the mixture was cooled to -30°C, the Grignard compound obtained by the above process was added, and the resulting mixture was stirred for 2 hours to complete the reaction.

This mixture was returned to room temperature, and extracted with toluene/hydrochloric acid. The organic layer was evaporated under reduced pressure. The obtained solid was purified by silica gel column chromatography to give phenyl thionobenzoate as a yellow solid (11.9 g, 55.5 mmol). The yield was 87%.

### Examples 2-19

Using the starting materials shown in Tables 1 and 2, the reaction was performed in the same manner as in Example 1. Tables 1 and 2 show the kinds and yields of the obtained products. The each Grignard compound shown in the Compound (2) column of the table was obtained by reacting magnesium with a halide serving as precursor of the each Grignard compound in the same manner as in Example 1. The numerical values shown in the Catalyst column of the table are the amounts of catalyst expressed in mol% relative to Compound (1). Example 18 is a comparative example wherein the reaction has been conducted in the absence of a catalyst.

**Table 1**

| Example | Compound (1) | Catalyst | Compound (2) | Solvent | Temp. °C | Product | Yield (%)* |
|---|---|---|---|---|---|---|---|
| 1 | 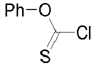 | Fe(acac)₃ 3mol% | 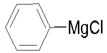 | THF | -30°C | 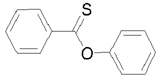 | 87 |
| 2 | 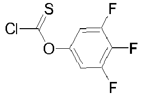 | Fe(acac)₃ 3mol% | 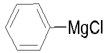 | THF | -30°C | 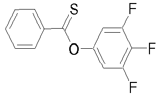 | 87 |
| 3 | 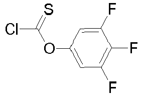 | Fe(acac)₃ 1mol% | 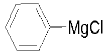 | THF | -30°C | 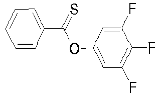 | 75* |
| 4 | 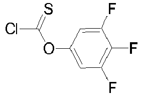 | FeCl₃ 3mol% | 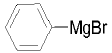 | THF | -30°C | 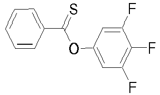 | 83* |
| 5 | 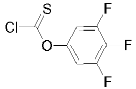 | NiCl₂(dpp p) 1mol% | 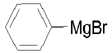 | THF | -30°C | 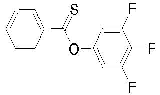 | 72* |
| 6 | 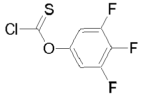 | CuCl₃ 100mol% | 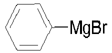 | THF | -30°C | 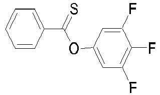 | 64* |
| 7 | 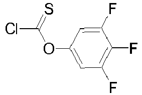 | CoCl₂ 1mol% | 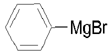 | THF | -30°C | 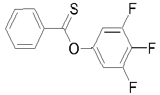 | 23* |
| 8 | 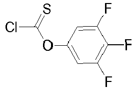 | Li₂CuCl₄ 5mol% | 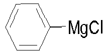 | THF | -30°C | 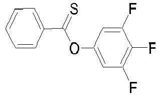 | 68* |
| 9 | 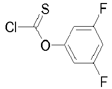 | Fe(acac)₃ 3mol% | 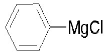 | THF | -30°C | 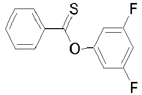 | 82* |
| 10 | 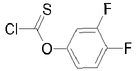 | Fe(acac)₃ 3mol% | 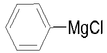 | THF | -30°C | 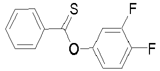 | 83* |

| | | | | | | | |
|---|---|---|---|---|---|---|---|
| * Determined by ¹⁹F NMR | | | | | | | |

**Table 2**

| Example | Compound (1) | Catalyst | Compound (2) | Solvent | Temp. °C | Product | Yield (%)* |
|---|---|---|---|---|---|---|---|
| 11 | 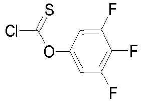 | Fe(acac)₃ 3mol% | 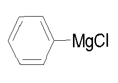 | THF | -30°C | 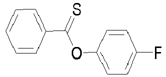 | 85* |
| 12 | 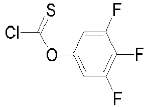 | Fe(acac)₃ 3mol% | 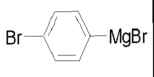 | THF | -30°C | 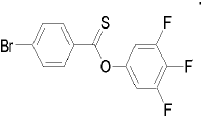 | 78* |
| 13 | 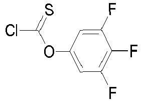 | Fe(acac)₃ 3mol% | 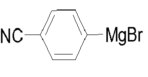 | THF | -30°C | 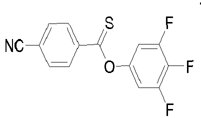 | 80* |
| 14 | 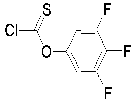 | Fe(acac)₃ 3mol% | 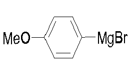 | THF | -30°C | 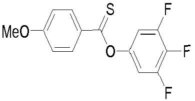 | 77* |
| 15 | 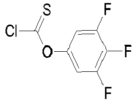 | Fe(acac)₃ 3mol% | 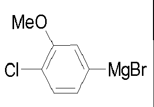 | THF | -30°C | 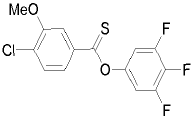 | 75* |
| 16 | 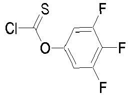 | Fe(acac)₃ 3mol% | 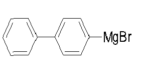 | THF | -30°C | 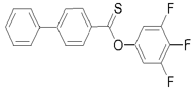 | 83 |
| 17 | 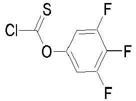 | Fe(acac)₃ 3mol% | 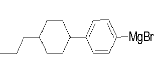 | THF | -30°C | 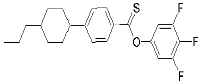 | 82 |
| 18 | 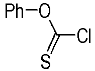 | no cat. | 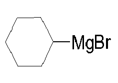 | THF | -30°C | 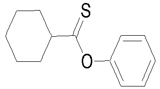 | 20 |
| 19 | 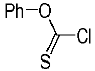 | Fe(acac)₃ 3mol% | 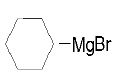 | THF | -30°C | 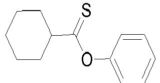 | 45 |

| | | | | | | | |
|---|---|---|---|---|---|---|---|
| * Determined by ¹⁹F NMR | | | | | | | |

### Example 20

Magnesium (1.6 g, 64.0 mmol), dibromoethane (0.6 g, 3.2 mmol), and cyclopentyl methyl ether (CPME) (10.0 mL) were added in a nitrogen atmosphere to a 50 ml three-necked flask equipped with a stirring bar and a thermometer. After bromocyclohexane (10.4 g, 64 mmol) was added, the mixture was stirred at 60°C for 1 hour to produce a Grignard compound.

Subsequently, phenyl chlorothionoformate (11.0 g, 64 mmol), a 0.1 M tetrahydrofuran solution of dilithium tetrachlorocuprate(II)(19 mL, 1.9 mmol), and THF (10 mL) were added in a nitrogen atmosphere to a 200 ml three-necked flask equipped with a stirring bar and a thermometer. After the mixture was cooled to -30°C, the Grignard compound obtained by the above process was added, and the resulting mixture was stirred for 2 hours to complete the reaction. This mixture was returned to room temperature, and extracted with toluene/hydrochloric acid. The organic layer was evaporated under reduced pressure. The obtained solid was purified by silica gel column chromatography to give cyclohexyl thionobenzoate as a yellow solid (10.1 g, 46 mmol). The yield was 72%.

### Examples 21-23

Using the starting materials shown in Table 3, the reaction was performed in the same manner as in Example 20. Table 3 shows the kinds and yields of the obtained products. The each Grignard compound shown in the Compound (2) column of the table was obtained by reacting magnesium with a halide serving as precursor of each Grignard compound in the same manner as in Example 20. Further, the numerical values shown in the Catalyst column of the table are the amounts of catalyst expressed in mol% relative to Compound (1).

**Table 3**

| Example | Compound (1) | Catalyst | Compound (2) | Solvent | Temp. °C | Product | Yield (%)* |
|---|---|---|---|---|---|---|---|
| 20 | 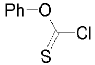 | Li₂CuCl₄ 3mol% | 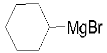 | CPME/ THF | -30°C | 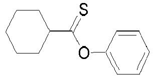 | 72 |
| 21 | 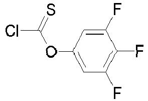 | Li₂CuCl₄ 3mol% | 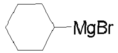 | CPME/ THF | -30°C | 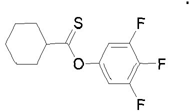 | 65 |
| 22 | 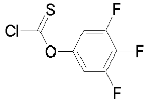 | Li₂CuCl₄ 3mol% | 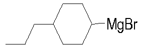 | CPME/ THF | -30°C | 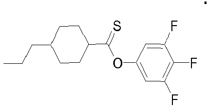 | 68* |
| 23 | 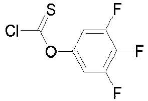 | Li₂CuCl₄ 3mol% | 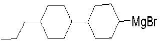 | CPME/ THF | -30°C | 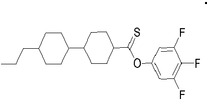 | 62 |

| | | | | | | | |
|---|---|---|---|---|---|---|---|
| * Determined by ¹⁹F NMR | | | | | | | |

## Claims

1. A process for producing an arylthionocarboxylic acid ester of formula (3):
R-C(=S)-O-Ar (3)
wherein Ar is optionally substituted aryl, and R is aryl or cycloalkyl, each optionally substituted; comprising reacting a halogenated thiocarbonyl compound of formula (1) :
X-C(=S)-O-Ar (1)
wherein Ar is as defined above and X is halogen, with a Grignard compound of the formula R-MgY (2) wherein R is as defined above and Y is halogen,
in the presence of at least one component selected from trivalent iron compounds, monovalent copper compounds, divalent copper compounds, divalent nickel compounds, divalent zinc compounds, and divalent cobalt compounds.

2. The process of claim 1, wherein the reaction is performed in an aprotic organic solvent.

3. A process for producing an arylthionocarboxylic acid ester of formula (3):
R-C(=S)-O-Ar (3)
wherein Ar is an optionally substituted aryl group and R is optionally substituted aryl or optionally substituted cycloalkyl;
the process comprising reacting a halide represented by Formula R-Y (4)wherein R is as defined above and Y is a halogen atom, with a magnesium compound,
and then reacting the reaction product with a halogenated thiocarbonyl compound of formula (1):
X-C(=S)-O-Ar (1)
wherein Ar is as defined above and X is a halogen atom, in the presence of at least one component selected from trivalent iron compounds, monovalent copper compounds, divalent copper compounds, divalent nickel compounds, divalent zinc compounds, and divalent cobalt compounds.

## Patentansprüche

1. Verfahren zur Herstellung eines Arylthioncarbonsäureesters der Formel (3):
R-C(=S)-O-Ar (3)
worin Ar gegebenenfalls substituiertes Aryl ist und R ein Aryl oder Cycloalkyl ist, das jeweils gegebenenfalls substituiert ist; umfassend das Reagieren einer halogenierten Thiocarbonylverbindung der Formel (1):
X-C(=S)-O-Ar (1)
worin Ar wie oben definiert ist und X Halogen ist, mit einer Grignard-Verbindung der Formel R-MgY (2), worin R wie oben definiert ist und Y Halogen ist,
in Gegenwart mindestens einer Komponente, ausgewählt aus dreiwertigen Eisenverbindungen, einwertigen Kupferverbindungen, zweiwertigen Kupferverbindungen, zweiwertigen Nickelverbindungen, zweiwertigen Zinkverbindungen und zweiwertigen Kobaltverbindungen.

2. Verfahren gemäß Anspruch 1, worin die Reaktion in einem aprotischen organischen Lösungsmittel durchgeführt wird.

3. Verfahren zur Herstellung eines Arylthioncarbonsäureesters der Formel (3):
R-C(=S)-O-Ar (3)
worin Ar eine gegebenenfalls substituierte Arylgruppe ist und R ein gegebenenfalls substituiertes Aryl oder ein gegebenenfalls substituiertes Cycloalkyl ist;
das Verfahren umfassend das Reagieren eines durch die Formel R-Y (4) dargestellten Halogenids, worin R wie oben definiert ist und Y ein Halogenatom ist, mit einer Magnesiumverbindung,
und anschließendes Reagieren des Reaktionsprodukts mit einer halogenierten Thiocarbonylverbindung der Formel (1) :
X-C(=S)-O-Ar (1)
worin Ar wie oben definiert ist und X ein Halogenatom ist, in Gegenwart mindestens einer Komponente, ausgewählt aus dreiwertigen Eisenverbindungen, einwertigen Kupferverbindungen, zweiwertigen Kupferverbindungen, zweiwertigen Nickelverbindungen, zweiwertigen Zinkverbindungen und zweiwertigen Kobaltverbindungen.

## Revendications

1. Procédé de production d'un ester d'acide arylthionocarboxylique de formule (3) :
R-C(=S)-O-Ar (3)
dans laquelle Ar est un aryle facultativement substitué, et R est un aryle ou un cycloalkyle, chacun facultativement substitué ; comprenant la réaction d'un composé thiocarbonyle halogéné de formule (1) :
X-C(=S)-O-Ar (1)
dans laquelle Ar est tel que défini ci-dessus et X est un halogène, avec un composé de Grignard de formule R-MgY (2) dans laquelle R est tel que défini ci-dessus et Y est un halogène,
en présence d'au moins un composant sélectionné parmi les composés de fer trivalent, les composés de cuivre monovalent, les composés de cuivre divalent, les composés de nickel divalent, les composés de zinc divalent, et les composés de cobalt divalent.

2. Procédé selon la revendication 1, dans lequel la réaction est réalisée dans un solvant organique aprotique.

3. Procédé de production d'un ester d'acide arylthionocarboxylique de formule (3) :
R-C(=S)-O-Ar (3)
dans laquelle Ar est un groupe aryle facultativement substitué et R est un aryle facultativement substitué ou un cycloalkyle facultativement substitué ;
le procédé comprenant la réaction d'un halogénure représenté par la formule R-Y (4), dans laquelle R est tel que défini ci-dessus et Y est un atome d'halogène, avec un composé de magnésium,
et ensuite la réaction du produit réactionnel avec un composé thiocarbonyle halogéné de formule (1) :
X-C(=S)-O-Ar (1)
dans laquelle Ar est tel que défini ci-dessus et X est un atome d'halogène, en présence d'au moins un composant sélectionné parmi les composés de fer trivalent, les composés de cuivre monovalent, les composés de cuivre divalent, les composés de nickel divalent, les composés de zinc divalent, et les composés de cobalt divalent.
